# EUROPEAN PATENT SPECIFICATION

(11) **EP 3 399 050 B1**
(45) Date of publication and mention of the grant of the patent: **18.03.2020**
(21) Application number: 17179602.2
(22) Date of filing: 04.07.2017
(51) Int. Cl.: C12Q 1/18, G01N 33/487

(54) **A TEST STRIP FOR MINIMAL INHIBITION CONCENTRATION (MIC) DETECTION**
TESTSTREIFEN FÜR DEN NACHWEIS EINER MINIMALEN HEMMKONZENTRATION
BANDE DE TEST POUR LA DÉTECTION D'UNE CONCENTRATION D'INHIBITION MINIMALE (MIC)

(30) Priority: 02.05.2017 TR 201706407
(43) Date of publication of application: 07.11.2018
(73) Proprietor: Kuleoglu, Mustafa Remzi, Ankara (TR); Kuleoglu, Enver Ali, Ankara (TR)
(72) Inventor: Kuleoglu, Mustafa Remzi, Ankara (TR); Kuleoglu, Enver Ali, Ankara (TR)
(74) Representative: Dericioglu, E. Korhan

(56) References cited:
- EP-B1- 2 480 682
- Anonymous: "Etest Antimicrobial Susceptibility Testing", , 31 December 2012 (2012-12-31), XP055437247, Retrieved from the Internet: URL:http://www.ilexmedical.com/files/E-tes t-Package-Insert/AST_WW.pdf [retrieved on 2017-12-21]
- Anonymous: "Antimicrobial Susceptibility Testing", , 31 December 2008 (2008-12-31), XP055437230, Retrieved from the Internet: URL:http://www.oxoid.com/pdf/uk/MICE-monog raph-march08.pdf [retrieved on 2017-12-21]

## Description

### Field of the Invention

The present invention relates to minimal inhibition concentration (MIC) detection test bands, which are produced by applying antibiotics on cotton, linen or silk fabrics, or fabrics of cotton-polymer mixture.

### Background of the Invention

Antimicrobial susceptibility tests are tests which are applied in order to detect in vitro activity of an antimicrobial agent against a specific bacteria species and mainly two methods are used namely, "diffusion" "and "dilution".

Dilution tests are applied to detect minimum concentration of an antimicrobial agent required to inhibit reproduction of a microorganism or to kill the same. Dilution tests are applied in two ways, namely "tube dilution" and "agar dilution".

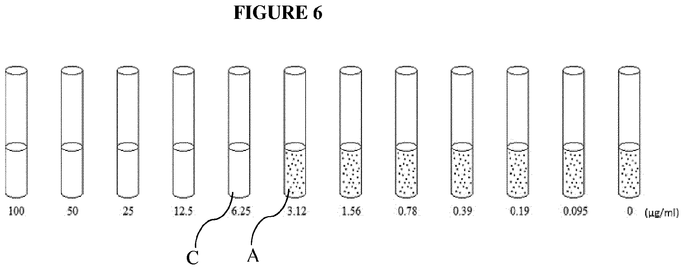

The publication of Irith Wiegand, Kai Hilpert and Robert E.W. Hancock in Centre for Microbial Diseases and Immunity Research (163-175) (2008) dated January 17, 2008 discloses that tube dilution method can be applied in two ways namely "macro" and "micro". In principle, both methods are the same but test tubes are used in macrodilution whereas "U" or "V" based "microplates" are used in microdilution. In tube dilution method, cation (calcium and magnesium) added Mueller-Hinton broth is used as the medium. The antibiotics to be tested are first prepared in their special solvents and then are diluted in this broth to a two times lower concentration. A standard inoculum of the microorganism is prepared and it is added to each tube containing various dilutions of the antimicrobial agent at equal amounts. Additionally, it is also added to the control tube which does not include antibiotic and which is the indicator of reproduction. A tube or a well, wherein bacteria are not inoculated but only the medium is disposed, is prepared as the control medium. The media are examined in terms of turbidity, which shows bacterial growth, after incubation of 16-20 hours at 37°C. The lowest drug concentration, which prevents bacterial growth and wherein there is no visible turbidity, is evaluated to be the minimum inhibitor concentration (MIC). The MIC values obtained as a result of the dilution tests provide information to the clinician about the antimicrobial drug concentration required to inhibit the microorganism causing the infection (Figure 6).

In a state-of-the-art application, the antibiotics were decreased from left to right and were respectively diluted in the broth, and an equal volume and equal concentration of microorganisms were added thereon. The tubes into which microorganisms were added were incubated at 37°C for 16-20 hours and the obtained results were evaluated. The antibiotic concentration in the tube, wherein microorganisms did not reproduce, was recorded as MIC value (6.25 µg/ml)^{[1, 2]}.

The invention disclosed in the United States patent document no. US47783758, known in the state of the art, relates to MIC tests used for in vitro diagnosis, and it is stated that the detection test band used is transparent, inert and has a nonporous surface. By means of this patent, instead of MIC detection carried out by tube dilution test, it was succeeded to inoculate on a band for the first time.

"Etest Antimicrobial Susceptibility Testing", 31 December 2012 (2012-12-31), retrieved from the internet:URL:http://www.ilexmedical.com/files/E-test-Package-Insert/ AST_WW.pdf [retrieved on 2017-12-21] discloses a MIC test band made from plastic.

"Antimicrobial Susceptibility Testing", 31 December 2008 (2008-12-31), retrieved from the internet:URL:http://www.oxoid.com/pdf/uk/MICE-monograph-march08.pdf [retrieved on 2017-12-21] discloses a MIC test band made from plastic polymer.

The MIC test bands of Oxoid Limited company mentioned in the publication titled "M.I.C.E valuators (M.I.C.E.) Simple, Convenient Method for Accurate MIC Values" in RAPIDMICROBIOLOGY dated 05/30/2008 are produced from polymer material. Furthermore, United States patent document no. US6010910A of the above mentioned company discloses that the bands in the test kits can be produced from plastic, cardboard or similar materials.

In the invention disclosed in the European patent document numbered EP2480682 B1 of Liofilchem Srl company, it is stated that the detection test bands used for MIC tests are produced from paper material. It is stated that by using bands produced from paper material, formation of air bubbles in the band material adhered onto the medium, wherein bacteria are inoculated, can be prevented and air contact of the bands with the environment can be enabled. Additionally, in the method of the state of the art, it is pressed on the band material with a forcep in order to prevent formation of air bubbles after placing the band material on the medium. Therefore, formation of air bubbles can be prevented independent from the material used.

The invention disclosed in Chinese Utility Model document no. CN2564584, another state-of-the-art application, relates to a detection plate test band made of a membrane used for detecting minimum antibacterial concentration of bacteriophages. The filtration-membrane band is fixed on a substrate, thus leading to a detection plate of the bacterial drug sensitivity test. In the said invention, two detections can be carried out at once including drug sensitivity and minimum antibacterial concentration (MIC). It is stated that the said invention is advantageous due to its low cost, simple and easy application, and reliability.

When compared with the applications in the state of the art; it is seen that minimal inhibition concentration (MIC) detection test band production, wherein bands made from cotton fabric, linen fabric, silk fabric or fabrics of cotton-polymer mixture, is advantageous in that the material used therein is easy to supply, has low cost, is produced from materials resistant against wear-out and tear, and allows easier and faster diffusion of the antibiotic material.

### Summary of the Invention

The present invention relates to a test band according to independent claim 1. Preferred embodiments are defined in the dependent claims.

### Detailed Description of the Invention

The "Test Band For MIC (Minimal Inhibition Concentration) Detection" developed to fulfill the objective of the present invention is illustrated in the accompanying figures wherein,

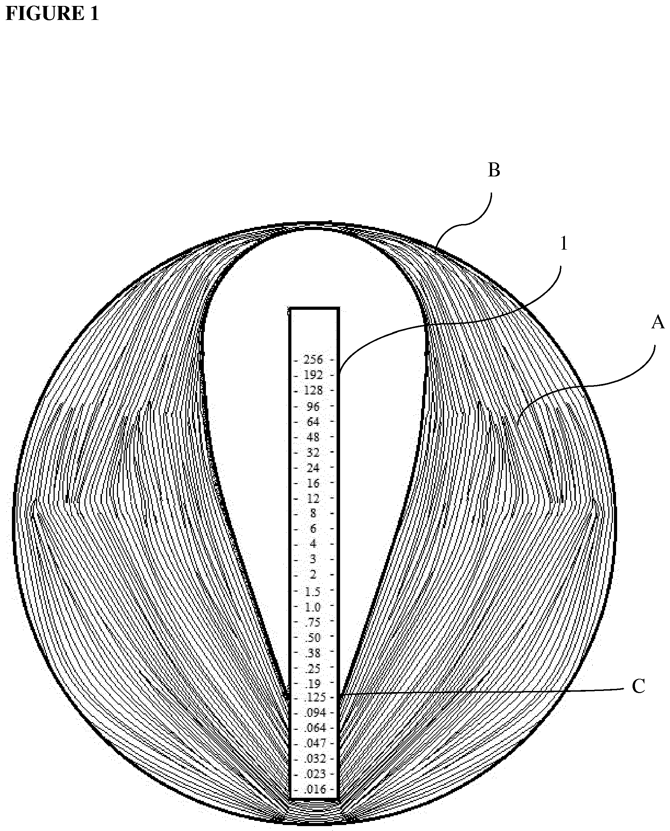

- **Figure 1.**: is a view of the test band for MIC (Minimal Inhibition Concentration) Detection in a petri dish.

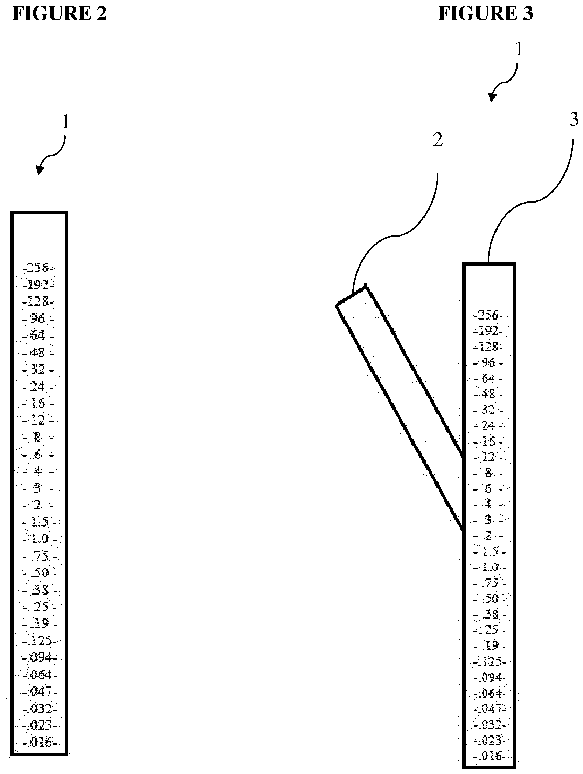

- **Figure 2.**: is a general view of the test band for MIC (Minimal Inhibition Concentration) Detection.
- **Figure 3.**: is a view of the layers of the test band for MIC (Minimal Inhibition Concentration) Detection.

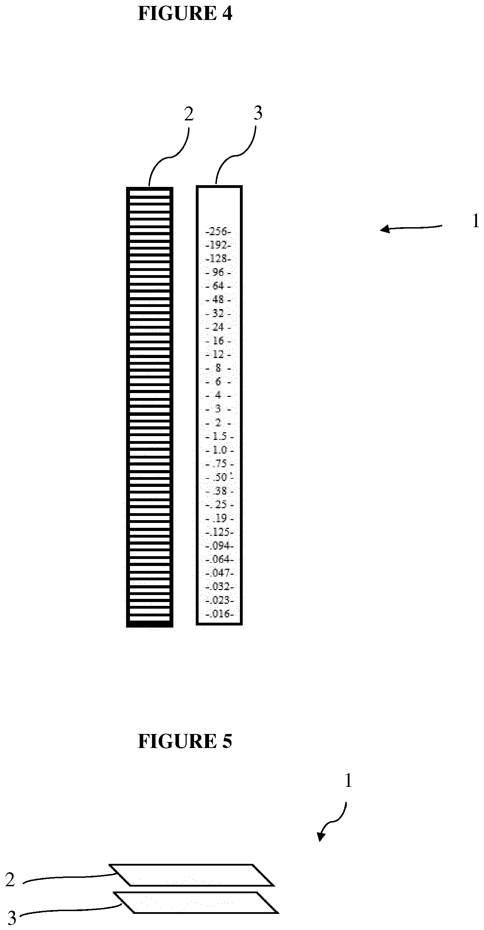

- **Figure 4.**: is an exploded view of the layers of the test band for MIC (Minimal Inhibition Concentration) Detection.
- **Figure 5.**: is an exploded view of the test band for MIC (Minimal Inhibition Concentration) Detection.
- **Figure 6.**: is a view of the tube dilution method application.

The components in the figures are given reference numbers as follows:
**1.** Test band for MIC (Minimal Inhibition Concentration) Detection
**2.** Interaction layer
**3.** Ruler layer

**A.** Microorganisms
**B.** Petri dish
**C.** MIC value

The inventive test band (1) for MIC (Minimal Inhibition Concentration) detection is placed into a petri dish (B) containing suitable agar medium such that its layer, on which a drug is inoculated, is contacted with the microorganisms (A) inoculated into the petri dish (B) with the purpose of detecting the measure of interaction (MIC value (C)) of the microorganisms (A) with the antibacterials (in antibiotic, antimycostatics or other biological fluids), by using an in vitro diagnosis device (IVD); and it is preferably in the form of a rectangular plate and is placed into the petri dish (B) such that its layer, on which antibiotic is inoculated, contacts the microorganisms (A) in the petri dish (B) and all parts thereof are ensured to be adhered by lightly pressing thereon via a penset; and **comprises**
- at least one interaction layer (2) which is in the form of a rectangular plate and is positioned so as to contact the microorganisms (A) in the petri dish (B),
- at least one ruler layer (3) which is in the same form and size with the interaction layer (2) and is connected to the interaction layer (2) from the face thereof that does not contact the microorganisms (A);
and is **characterized by**
- the interaction layer (2); on which an antibiotic, whose interaction with microorganisms (A) will be examined, is applied on the surface thereof that contacts the microorganisms (A) by gradually decreasing amounts between the short sides of its rectangular form; and which is made from cotton fabric, linen fabric, silk fabric or fabrics of cotton - polymer mixture,
- the ruler layer (3), which, in order to figure out the antibiotic concentration applied onto the interaction layer (2), includes numbers denoting the concentration value and lines denoting the positions corresponding to these concentrations.

In one embodiment of the invention, the ruler layer (3) is produced by using cotton fabric. Accordingly, in this embodiment of the invention, the ruler layer (3) and the interaction layer (2) are produced together as a single piece and are not connected. The test bands according to the invention preferably do not have air permeability.

In one embodiment of the invention, the polymer used in the structures comprised of cotton fabric and polymer combination is preferred to be polypropylene and ethylene butylacrylate copolymer.

In one embodiment of the invention, the interaction layer (2) includes chambers, which enable the inoculated antibiotic to easily diffuse to the medium, on the surface thereof contacting the microorganisms (A).

In a different embodiment of the invention, a polymer-cotton fabric mixture, whose absorption capacity is higher than that of the paper applications in the state of the art, is used as the interaction layer (2). In this sense, synthetic fabrics having 50% or more cotton content are also used as cotton fabric. As an alternative to cotton fabric; cotton cloth, cotton and polyester mixture cloth, linen cloth or silk cloth are also used in the scope of the invention, as defined by the claims.

In a different embodiment of the invention, the ruler layer (3) is produced from aluminum, plastic or paper sticker.

In one embodiment of the invention, the ruler layer (3) is in the form of a sticker and is adhered onto the surface of the interaction layer (2) that does not contact the microorganisms (A). After the ruler layer (3) is produced, the numerical values and lines, which enable to read measurements, are provided on the surface thereof by printing in the printing press.

The MIC test band (1) of the present invention enables both inoculation of the drug and the inoculated drug to adhere to the surface and remain there evenly. Drug inoculation is performed after the ruler layer (3) and the interaction layer (2) are connected and thus the MIC test band (1) structure of the present invention is formed. The drug inoculation on the interaction layer (2) is performed between the two short sides of the interaction layer (2) in a decreasing manner, i.e. gradient manner, in accordance with the concentration values in the ruler layer (3). The MIC test bands according to the present invention are preferably produced in the form of a rectangular band. This way, it can be easily placed on a medium such as in a petri dish (B) in which the microorganisms are inoculated, and the intersection point of the interaction between the drug and the bacteria can be detected more specifically by means of the numbers on the ruler. This way, being able to express the minimal antibiotic concentration, at which microorganism-antibiotic interaction takes place, denoted as MIC value, as a numeric value facilitates the procedures for the user.

The MIC test bands of the present invention can be stored within the range of -20 to 8°C. On the other hand, MIC test bands comply with two standard values accepted worldwide, namely CLSI (Clinic and Laboratory Standards Institute) in the United States and EUCAST (European Committee on Antimicrobial Susceptibility Testing) in European Union.

Gradually decreasing amounts of antibiotic are added to the test bands produced by using cotton fabric, linen fabric, silk fabric or fabrics of cotton-polymer mixture; and a ruler is printed on the layer of the test band, on which a drug (antibiotic) is not inoculated, and which is produced from aluminum material, in order to be able to evaluate the experiment result. The minimum antibiotic concentration (mg/l), at which bacterial growth is inhibited, of the ruler arranged according to the MIC value ranges known in the literature is defined as MIC value.

Using cotton fabric, linen fabric, silk fabric or fabrics of cotton-polymer mixture for their production enabled to obtain bands, which are more resistant against tearing and breaking, more advantageous in terms of cost and easy to supply, and which allow the antibiotic material to diffuse more easily and rapidly. Furthermore, it is observed that the interaction between the antibiotic and bacteria can be prevented from getting affected by the air in the outer environment by using bands not having air permeability.

In another embodiment of the invention, the layer of the test bands, on which drug is not inoculated and a ruler is printed, is produced from a plastic or paper material.

In another embodiment of the invention, the ruler layer is produced by using fabric material.

### REFERENCES

[1]. Bauer J.D., Ackerman P.G., Toro G., Clinical labroratory methods, 8th edition, The C.V. Mosby Company, St. Louis, USA 1974; 661-662
[2].Bauer J.D., Clinical laboratory methods, 9th edition, The C.V. Mosby Company, St. Louis, USA 1982 ; 883-884

## Claims

1. A test band (1) for Minimal Inhibition Concentration (MIC) detection suitable for being placed into a petri dish (B), comprising
- an interaction layer (2); which is in the form of a rectangular plate and which may be positioned so as to contact microorganisms (A) in the petri dish (B) and on which an antibiotic is applied, on the surface thereof for contacting the microorganisms (A), in gradually decreasing amounts between the short sides of its form of a rectangular plate;
- a ruler layer (3), which has the same form and size as the interaction layer (2) and is connected to the interaction layer (2) from the face thereof that is not for contacting the microorganisms (A) and which, in order to be able to figure out the antibiotic concentration applied onto the interaction layer (2), includes numbers denoting a value of the antibiotic concentration and lines denoting the positions corresponding to these antibiotic concentrations;
**characterized in that** the interaction layer is made from cotton fabric, linen fabric, silk fabric or fabrics of cotton-polymer mixture.

2. A test band (1) for MIC detection according to Claim 1, **characterized in that** the ruler layer (3) is made from cotton fabric and is formed as a single piece with the interaction layer (2).

3. A test band (1) for MIC detection according to Claim 1, **characterized in that** the interaction layer (2) does not have air permeability.

4. A test band (1) for MIC detection according to Claim 1, **characterized in that** the polymer of the fabrics of cotton-polymer mixture is polypropylene and ethylene butylacrylate copolymer.

5. A test band (4) for MIC detection according to Claim 4, **characterized in that** the fabrics of cotton-polymer mixture are synthetic fabrics having 50% or more cotton content.

6. A test band (1) for MIC detection according to Claim 1, **characterized in that** the ruler layer (3) is made from aluminum, plastic or paper material.

7. A test band (1) for MIC detection according to Claim 1, **characterized in that** the numbers and lines are printed numbers and lines.

8. A test band (1) for MIC detection according to Claim 1, **characterized in that** the ruler layer (3), is in the form of a sticker and adhered and fixed on the interaction layer (2).

## Patentansprüche

1. Testband (1) zur Erfassung der Minimal-Inhibitions-Konzentration (MIC), die zum Einbringen in eine Petrischale (B) geeignet ist, umfassend
eine Interaktionsschicht (2); die die Form einer rechteckigen Platte hat und die so positioniert werden kann, dass sie mit Mikroorganismen (A) in der Petrischale (B) in Kontakt kommt, und auf deren Oberfläche ein Antibiotikum aufgetragen wird, um mit den Mikroorganismen (A) in graduell abnehmenden Mengen zwischen den kurzen Seiten ihrer Form einer rechteckigen Platte in Kontakt zu kommen;
eine Linienschicht (3), die die gleiche Form und Größe wie die Interaktionsschicht (2) hat und mit der Interaktionsschicht (2) von deren nicht für den Kontakt mit den Mikroorganismen (A) dienende Fläche aus verbunden ist und die, um die auf die Interaktionsschicht (2) aufgebrachte Antibiotikakonzentration ermitteln zu können, Zahlen, die einen Wert der Antibiotikakonzentration bezeichnen, und Linien, die die diesen Antibiotikakonzentrationen entsprechenden Positionen bezeichnen, enthält; **dadurch gekennzeichnet, dass** die Interaktionsschicht aus Baumwollgewebe, Leinengewebe, Seidengewebe oder Gewebe aus Baumwoll-Polymer-Mischung hergestellt ist.

2. Testband (1) zur Erfassung der MIC nach Anspruch 1, **dadurch gekennzeichnet, dass** die Linienschicht (3) aus Baumwollgewebe besteht und einstückig mit der Interaktionsschicht (2) gebildet ist.

3. Testband (1) zur Erfassung der MIC nach Anspruch 1, **dadurch gekennzeichnet, dass** die Interaktionsschicht (2) keine Luftdurchlässigkeit aufweist.

4. Testband (1) zur Erfassung der MIC nach Anspruch 1, **dadurch gekennzeichnet, dass** das Polymer der Gewebe aus Baumwoll-Polymer-Mischung Polypropylen und Ethylen-Butylacrylat-Copolymer ist.

5. Testband (4) zur Erfassung der MIC nach Anspruch 4, **dadurch gekennzeichnet, dass** die Gewebe aus Baumwoll-Polymer-Mischung synthetische Gewebe mit 50% oder mehr Baumwollanteil sind.

6. Testband (1) zur Erfassung der MIC nach Anspruch 1, **dadurch gekennzeichnet, dass** die Linienschicht (3) aus Aluminium-, Kunststoff- oder Papiermaterial hergestellt ist.

7. Testband (1) zur Erfassung der MIC nach Anspruch 1, **dadurch gekennzeichnet, dass** die Zahlen und Zeilen aufgedruckte Zahlen und Zeilen sind.

8. Testband (1) zur Erfassung der MIC nach Anspruch 1, **dadurch gekennzeichnet, dass** die Linienschicht (3) in Form eines Aufklebers ist und auf die Interaktionsschicht (2) aufgeklebt und fixiert ist.

## Revendications

1. Bande de test (1) pour la détection de la Concentration Minimale d'Inhibition (MIC) adaptée pour être placée dans une boîte de Petri (B), comprenant
une couche d'interaction (2) ; qui a la forme d'une plaque rectangulaire et qui peut être positionnée de manière à entrer en contact avec les micro-organismes (A) dans la boîte de Pétri (B) et sur la surface de laquelle un antibiotique est appliqué pour entrer en contact avec les micro-organismes (A) en quantités progressivement décroissantes entre les petits côtés de sa forme de plaque rectangulaire ;
une couche de règle (3), qui a la même forme et la même taille que la couche d'interaction (2) et est connecté à la couche d'interaction (2) à partir de la face de celle-ci qui n'est pas destinée à entrer en contact avec les micro-organismes (A) et qui, afin de pouvoir déterminer la concentration d'antibiotiques appliquée sur la couche d'interaction (2), comprend des nombres indiquant une valeur de la concentration d'antibiotiques et des lignes indiquant les positions correspondant à ces concentrations d'antibiotiques ; **caractérisé en ce que** la couche d'interaction est faite de tissu de coton, de tissu de lin, de tissu de soie ou de tissu de mélange coton-polymère.

2. Bande de test (1) pour la détection des MIC selon la revendication 1, **caractérisée en ce que** la couche de règle (3) est faite de tissu de coton et est formée d'une seule pièce avec la couche d'interaction (2).

3. Bande de test (1) pour la détection des MIC selon la revendication 1, **caractérisée en ce que** la couche d'interaction (2) n'a pas de perméabilité à l'air.

4. Bande de test (1) pour la détection des MIC selon la revendication 1, **caractérisée en ce que** le polymère des tissus du mélange coton-polymère est le polypropylène et le copolymère éthylène-butylacrylate.

5. Bande de test (4) pour la détection des MIC selon la revendication 4, **caractérisée en ce que** les tissus de mélange coton-polymère sont des tissus synthétiques ayant une teneur en coton de 50% ou plus.

6. Bande de test (1) pour la détection des MIC selon la revendication 1, **caractérisée en ce que** la couche de règle (3) est en aluminium, en plastique ou en papier.

7. Bande de test (1) pour la détection des MIC selon la revendication 1, **caractérisée en ce que** les nombres et les lignes sont des nombres et des lignes imprimés.

8. Bande de test (1) pour la détection des MIC selon la revendication 1, **caractérisée en ce que** la couche de règle (3) se présente sous la forme d'un autocollant et est collée et fixée sur la couche d'interaction (2).
